# EUROPEAN PATENT APPLICATION

(11) **EP 4 679 100 A1**
(43) Date of publication of application: **14.01.2026**
(21) Application number: 24770548.6
(22) Date of filing: 29.02.2024
(51) Int. Cl.: G01N 35/10, G01N 35/00, G01N 35/08, G01N 37/00

(54) **SPECIMEN ANALYZER, SPECIMEN ANALYSIS SYSTEM, AND SPECIMEN DISPENSING METHOD**

(30) Priority: 10.03.2023 JP 2023038002
(71) Applicant: HORIBA, Ltd., Minami-ku Kyoto-shi Kyoto 601-8510 (JP)
(72) Inventor: HIRATA Katsuki, Kyoto-shi, Kyoto 601-8510 (JP); SANO Tomoki, Kyoto-shi, Kyoto 601-8510 (JP)
(74) Representative: Isarpatent
(86) International application number: PCT/JP2024/007536
(87) International publication number: WO 2024/190437

(57) **Abstract**

A specimen analysis apparatus includes a specimen measurement cell and a dispensing mechanism that dispenses a specimen. The dispensing mechanism dispenses the specimen to the specimen measurement cell and to a test device held in a different place from the specimen measurement cell.

## Description

### Technical Field

The present invention relates to a specimen analysis apparatus for analyzing a specimen such as blood, a specimen analysis system including such a specimen analysis apparatus, and a specimen dispensing method for dispensing a specimen.

### Background Art

Conventionally, an apparatus is known in which a blood collection tube containing blood is set and that measures the total counts of different types of hemocytes (also called blood count) in the blood and the CRP (C-reactive protein) in it (e.g., Patent Document 1).

### Citation List

### Patent Literature

Patent Document 1: JP 2014-215210A

### Summary of Invention

### Technical Problem

Depending on for what measurement item blood is analyzed, a method is also known that achieves the measurement using a dedicated chip (hereinafter, referred to as test device). More specifically, the dedicated chip is spotted with blood so that it reacts with a reagent in the chip so that measurement for a measurement item is carried out by a detection mechanism based on a measurement principle. Carrying out measurement by various methods is effective because an operator (a medical professional) can then judge the condition of a test subject in a multifaceted manner (from multiple angles) based on different measurement results. However, obtaining measurement results using different measurement methods requires an operator to carry out different tasks such as setting a blood collection tube in an apparatus for measuring blood count and the like, and spotting a test device with blood. These tasks demand a lot of time from operators with minute-to-minute schedules.

The present invention is made to cope with the above inconvenience and its object is to provide a specimen analysis apparatus, a specimen analysis system, and a specimen dispensing method that allow multifaceted testing of a specimen such as blood while reducing the burden of tasks on operators.

### Solution to Problem

According to one aspect of the present invention, a specimen analysis apparatus includes a specimen measurement cell and a dispensing mechanism that dispenses a specimen. The dispensing mechanism dispenses the specimen to the specimen measurement cell and to a test device held in a different place from the specimen measurement cell.

According to another aspect of the present invention, a specimen analysis system includes the specimen analysis apparatus described above and a centrifugal mechanism that centrifugally separates, by rotating the test device, the components of the specimen with which the test device is spotted.

According to still another aspect of the present invention, a specimen dispensing method includes a loading step of setting the test device at a different place from the specimen measurement cell and a dispensing step of dispensing the specimen to the test device using the dispensing mechanism that dispenses the specimen to the specimen measurement cell.

### Advantageous Effects of Invention

The present invention allows multifaceted testing of a specimen while reducing the burden of tasks on operators.

### Brief Description of Drawings

[FIG. 1] is a perspective view showing an exterior configuration of a blood analysis apparatus that constitutes a blood analysis system according to one embodiment of the present invention.
[FIG. 2] is an illustrative diagram schematically showing the internal structure of the blood analysis apparatus.
[FIG. 3] is a perspective view showing the exterior appearance of a centrifugal analyzer.
[FIG. 4] is a sectional view schematically showing the configuration of the centrifugal mechanism in the centrifugal analyzer.
[FIG. 5] is a plan view of a chip as a test device.
[FIG. 6] is a perspective view, as seen from above, of a device holding portion with the test device held in it.
[FIG. 7] is a perspective view of the device holding portion as seen from below.
[FIG. 8] is a perspective view of a cover of the device holding portion as seen from below.
[FIG. 9] is an illustrative diagram schematically showing how the chip is put into and taken out of the device holding portion.
[FIG. 10] is a plan view, as seen from above, of the device holding portion in the blood analysis apparatus.
[FIG. 11] is a perspective view showing another configuration of the device holding portion.
[FIG. 12A] is a perspective view of the chip held in a first adapter.
[FIG. 12B] is an exploded perspective view of the first adapter and the chip.
[FIG. 13A] is a perspective view of a reagent kit held in a second adapter.
[FIG. 13B] is an exploded perspective view of the second adapter and the reagent kit.
[FIG. 14] is a plan view showing side by side how the device holding portion holds the chip via the first adapter and holds the reagent kit via the second adapter.
[FIG. 15] is a perspective view of another configuration of the blood analysis system.
[FIG. 16] is a front view of yet another configuration of the blood analysis system.
[FIG. 17] is a plan view schematically showing another configuration of the blood analysis apparatus.
[FIG. 18] is a sectional view schematically showing the procedure for ejecting blood from a suction tube to the spotted portion of the chip.
[FIG. 19] is a sectional view schematically showing a preferred procedure for ejecting blood to the spotted portion.

### Description of Embodiments

Now, an illustrative embodiment of the present invention will be described with reference to the drawings. Hereinafter, as one example of a specimen, blood (whole blood) is taken and, as one example of a specimen analysis apparatus, a blood analysis apparatus for analyzing blood is taken.

### [1. Overview of Blood Analysis Apparatus]

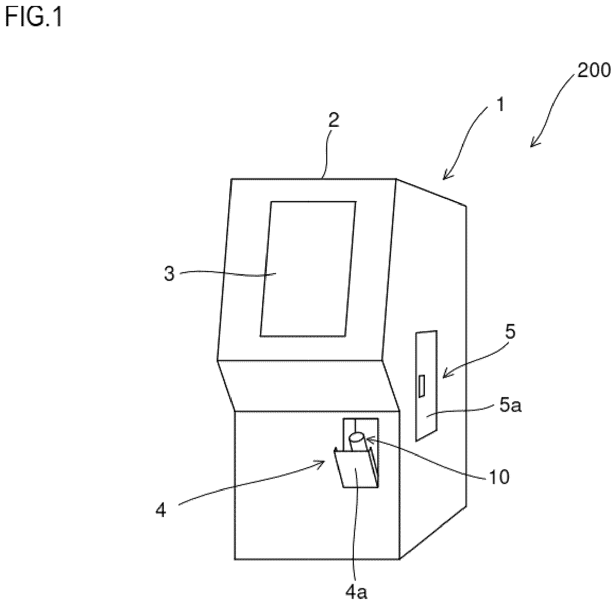

Fig. 1 is a perspective view showing an exterior configuration of a blood analysis apparatus 1 according to the embodiment. The blood analysis apparatus 1 includes a display 3 in top part of the front face of an apparatus body 2. The display 3 comprises a liquid crystal display device fitted with, for example, a touch panel; it displays analysis results on blood and the like and receives input of various kinds of information by an operator (e.g., a medical professional).

In a bottom part of the apparatus body 2, a specimen container loading portion 4 is provided. Opening a swinging lid 4a of the specimen container loading portion 4, setting in it a specimen container 10 containing a blood specimen (hereinafter, referred to simply as blood), and closing the swinging lid 4a permits the specimen container 10 to be loaded in the apparatus body 2. The specimen container 10 is, for example, a blood collection tube. Usable as a blood collection tube is a blood collection tube with or without a lid (closed type or open type, respectively).

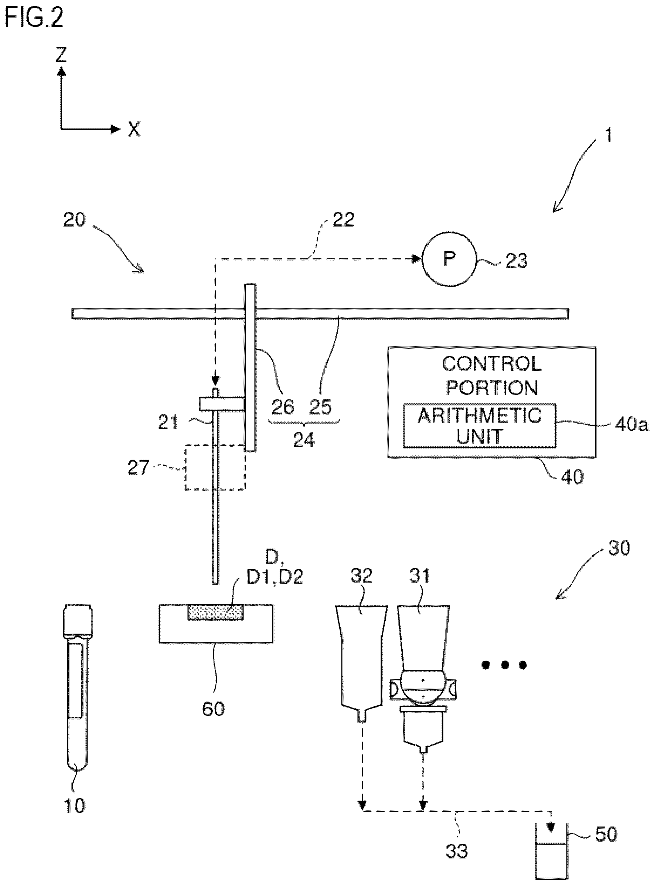

In a side part of the apparatus body 2, a reagent container loading portion 5 is provided. Sliding open a sliding lid 5a of the reagent container loading portion 5 allows replenishment with a reagent for use in blood analysis (e.g., a reagent for immunoassay measurement level) and insertion and extraction of a chip D1 (see Fig. 2 and the like) as a test device, which will be described later.

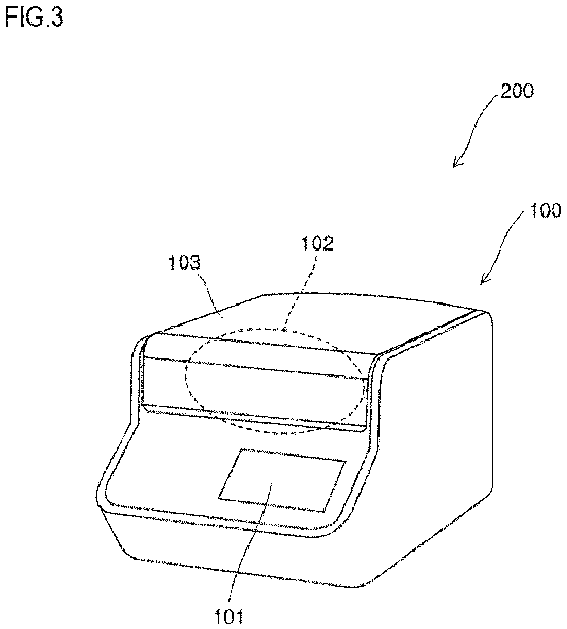

The blood analysis apparatus 1 described above can be used in combination with a centrifugal analyzer 100 (see Fig. 3), which will be described later. In that case, the blood analysis apparatus 1 cooperates with the centrifugal analyzer 100 to constitute a blood analysis system 200 (specimen analysis system).

### [2. Internal Structure of Blood Analysis Apparatus]

Fig. 2 is an illustrative diagram schematically showing the internal structure of the blood analysis apparatus 1. For convenience's sake, the following description uses directions defined as follows: the direction along which a suction tube 21 is moved by a horizontal movement mechanism 25, which will be described later, is direction X; the direction along which the suction tube 21 is moved by a vertical movement mechanism 26, which will be described later, is direction Z; and the direction perpendicular to directions X and Z is direction Y. As seen from in front (the side facing the specimen container loading portion 4) of the blood analysis apparatus 1 shown in Fig. 1, directions X, Y, and Z in Fig. 2 respectively correspond to the front-rear, left-right, and top-bottom directions of the blood analysis apparatus 1 in Fig. 1. In addition, along each of directions X, Y, and Z, one side is the positive side (positive direction) and the other side is the negative side (negative direction). In each diagram, the direction pointing from the tail to the head of an arrow indicating a direction is the positive direction. Note that all these definitions of directions are only for ease of discussion and are not meant to limit any directions in actual implementation.

The blood analysis apparatus 1 includes, a dispensing mechanism 20 that dispenses blood as a specimen, a blood analysis portion 30 that analyzes the dispensed blood by mixing it with a reagent, and a control portion 40. The control portion 40 comprised, for example, a central arithmetic processing device (computer) called a CPU (central processing unit) and controls the operation of different parts of the blood analysis apparatus 1.

The dispensing mechanism 20 has the suction tube 21. The suction tube 21 is a tube for sucking the blood contained in the specimen container 10 and then ejecting the sucked blood to a measurement cell 31, which will be described later, or the like. The suction tube 21 can be a nozzle or a needle. The nozzle has, in its bottom face at the bottom end of the tube, a hole for suction or ejection of blood and is used, for example, when the specimen container 10 is a blood collection tube of an open type. The needle tapers toward its lower end and has, in the side face of its bottom end, a hole for suction or ejection of blood. The needle is used, for example, when the specimen container 10 is a blood collection tube of a closed type and penetrates the lid of the blood collection tube to suck the blood inside the blood collection tube.

A base end part of the suction tube 21 is connected via a first pipe 22, indicated by a broken line, and an electromagnetic valve device (not shown) to a quantitative syringe 23. The control portion 40 drives the quantitative syringe 23 and the electromagnetic valve device, so that blood is sucked and ejected by the suction tube 21.

The dispensing mechanism 20 has a probe unit 24 as a movement mechanism. The probe unit 24 is configured to include the horizontal movement mechanism 25 and the vertical movement mechanism 26. The horizontal movement mechanism 25 and the vertical movement mechanism 26 each comprise, for example, a feed screw mechanism. The feed screw mechanism rotates a feed screw with a stepping motor to move an engaging portion that engages with the feed screw. Thus, coupling the engaging portion of the horizontal movement mechanism 25 to the vertical movement mechanism 26, holding the suction tube 21 with the engaging portion of the vertical movement mechanism 26, and driving the stepping motor permits the suction tube 21 to move horizontally and vertically. That is, the probe unit 24 can move the suction tube 21 along directions X and Z. Note that the horizontal movement mechanism 25 and the vertical movement mechanism 26 can be configured, for example, to drive an endless belt with a drive roller or the like to move the suction tube 21.

If the measurement item is CRP, the dispensing mechanism 20 dispenses a reagent for CRP measurement in a manner similar to that for blood.

The blood analysis portion 30 analyzes blood by performing hemocyte counting, immunoassay, and the like. The blood analysis portion 30 has the measurement cell 31 that receives the blood sucked from the specimen container 10 and ejected to it by the suction tube 21. The measurement cell 31 is a measurement chamber that is fixed inside the blood analysis apparatus 1 and stores blood. The inside of the measurement cell 31 is washed with predetermined timing after every test and can be used repeatedly. The measurement cell 31 is provided so as to correspond to the target to be counted, such as erythrocytes, leukocytes (including subcategories of leukocytes), CRP, and the like. The measurement of CRP can be conducted as necessary.

In the measurement cell 31 for counting the number of hemocytes, a counting device is provided. The counting device can take one of various measurement methods depending on the type of hemocyte to be counted, such as an impedance method, flow cytometry, a focused flow impedance method, and the like. The control portion 40 processes the measurement data acquired by the counting device to map a frequency distribution or the like. The processing of the measurement data is carried out by an arithmetic unit 40a included in the control portion 40. Thus, the blood analysis apparatus 1 includes the measurement cell 31 (a specimen measurement cell) for analyzing a specimen (blood). Note that the counting device just mentioned can be provided outside the measurement cell 31 and connected to the measurement cell 31.

Near a chamber for CRP measurement, a plurality of reagent containers (not shown) are provided. The reagent containers are loaded with a hemolysis reagent, a buffer, an anti-human CRP sensitive latex immunology reagent, and the like respectively. When CRP measurement is performed, the reagents are sucked from the reagent containers by the suction tube 21 with predetermined timing and ejected to the corresponding measurement cell 31.

The blood analysis portion 30 has a washing cell 32. When the suction tube 21 is washed by a washing device 27, the washing cell 32 receives washing liquid dropped from above. If unnecessary blood is ejected from the suction tube 21, the washing cell 32 also receives it.

The washing device 27 is supported on the probe unit 24 described above and thus moves in the horizontal direction as the suction tube 21 moves along the horizontal direction by being driven by the probe unit 24. The suction tube 21 moves along the vertical direction relative to the washing device 27 by being driven by the probe unit 24.

To lower end parts of the measurement cell 31 and the washing cell 32 described above, a discharging tube 33, indicated by a broken line, is connected. The waste liquid in the cells is fed via an electromagnetic valve device (not shown) to a waste liquid container 50 by being driven by a discharging portion (not shown). The discharging portion just mentioned comprises a discharge syringe or a discharge pump.

The control portion 40 drives the probe unit 24 to move the suction tube 21 along directions X and Z, and this permits the suction tube 21 to move into and out of each of the specimen container 10, the measurement cell 31, and the reagent container. Further, the control portion 40 drives the quantitative syringe 23 to permit the suction tube 21 to suck blood from the specimen container 10 and to eject it to the measurement cell 31. The same applies to the reagent for CRP measurement; driving a dedicated syringe permits the suction tube 21 to suck the reagent for CRP measurement from the reagent container and to eject it to the measurement cell 31. These operations allow blood analysis (hemocyte counting and immunoassay) in the blood analysis portion 30.

The blood analysis apparatus 1 according to the embodiment further includes a device holding portion 60. The device holding portion 60 holds a test device D. The device holding portion 60 is provided at a position apart from the measurement cell 31 along direction X. Specifically, the device holding portion 60 is provided at the negative side of the measurement cell 31 along direction X. The test device D is a device that is used in analysis on another testing apparatus and that is replaceable (removably mounted) with respect to it for each test. Such a test device D need only be a device that stores a specimen; it can be, for example, a chip D1 or a reagent kit D2, which will be described later, and can be the one in the form of a cartridge, a cassette, or the like. After being spotted with a specimen, the test device D is moved to a different place for measurement. The device holding portion 60 that holds the test device D will be described in detail later.

The control portion 40 drives the probe unit 24 to move the suction tube 21 having sucked blood from the specimen container 10 along direction X and then move the suction tube 21 downward above the test device D; this permits the test device D to be spotted with the blood. After being spotted, the test device D can be taken out of the blood analysis apparatus 1 and be set in another testing apparatus for testing on it.

In this way, the dispensing mechanism 20 dispenses blood to the measurement cell 31 and to the test device D held (by the device holding portion 60) at a different place from the measurement cell 31. This allows both blood testing (e.g., hemocyte counting) on the blood analysis apparatus 1 (in the measurement cell 31) and testing on another testing apparatus. This saves the operator in testing on another testing apparatus described above, the task of manually spotting the test device D with blood or manually fitting it with an accessory part such as a capillary. As a result, it is possible to perform various tests (multifaceted testing) while reducing the burden of tasks on operators.

Specifically, providing the blood analysis apparatus 1 with the device holding portion 60 allows effective use of the existing configuration (e.g., the suction tube 21 and the probe unit 24) of the blood analysis apparatus 1 for the test device D to be spotted with blood.

A specimen dispensing method according to the embodiment includes a loading step of (an operator's) setting the test device D at a different place (e.g., the device holding portion 60) from the measurement cell 31 and a dispensing step of dispensing a specimen to the test device D described above using the dispensing mechanism 20 that dispenses a specimen to the measurement cell 31. This makes it possible to perform various tests multifacetedly while reducing the burden of tasks on operators.

### [3. Centrifugal Analyzer]

Now, before a detailed description of the device holding portion 60 mentioned above, a centrifugal analyzer that analyzes blood using the test device D and a chip D1 as one example of the test device D will be described.

Fig. 3 is a perspective view showing the exterior appearance of the centrifugal analyzer 100. The centrifugal analyzer 100 is a specimen testing apparatus that is placed outside the blood analysis apparatus 1 (see Fig. 1). On the front face of the body of the centrifugal analyzer 100, a display 101 fitted with a touch panel is provided. The display 101 displays analysis results on blood and the like and receives input of various kinds of information by an operator.

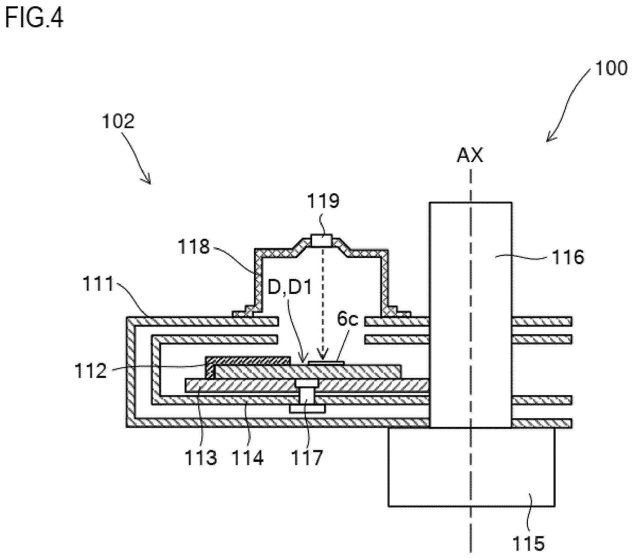

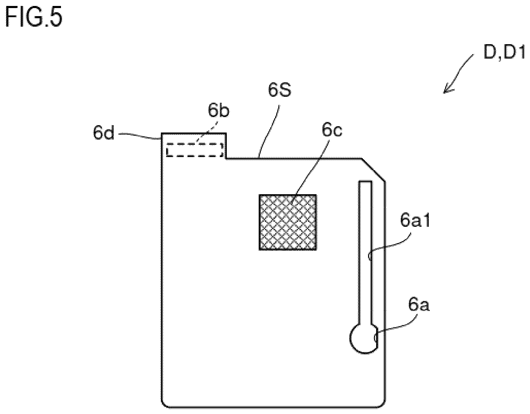

The centrifugal analyzer 100 has a centrifugal mechanism 102 in it. The centrifugal mechanism 102, by rotating the chip D1, centrifugally separates the components of the blood with which the chip D1 as the test device D is spotted (see Figs. 4 and 5). The chip D1 is spotted with blood inside the blood analysis apparatus 1 as described above. The chip D1 having been spotted with the blood is taken out of the blood analysis apparatus 1 and is set in the centrifugal mechanism 102. The chip D1 can be set in the centrifugal mechanism 102 with a lid 103 provided at the top of the body of centrifugal analyzer 100 swung open.

Fig. 4 is a sectional view schematically showing the configuration of the centrifugal mechanism 102. The centrifugal mechanism 102 has a measuring chamber 111. Inside the measuring chamber 111, the chip D1 is held by being pressed against a placement base 113 by a holder 112.

In the measuring chamber 111, a rotary table 114 is also provided. The rotary table 114 is rotated about a rotation axis AX by a motor 115. The placement base 113 is coupled to a driving force switching mechanism 116 having gears and cams. The driving force switching mechanism 116 switches the transmission of the driving force of the motor 115 to the placement base 113. This turns on or off the rotation of the placement base 113, and switches the direction of the centrifugal force acting on the chip D1 during the rotation of the rotary table 114. In this way, it is possible to control the direction of the blood flowing through a micro-passage in the chip D1.

The placement base 113 described above rotates (rotates on its axis) about a first planetary shaft 117 fitted to the rotary table 114. The first planetary shaft 117 is located away from the rotation axis AX of the rotary table 114 in the radial direction parallel to the rotation axis AX. In this way, by being driven by the motor 115, the placement base 113 can rotate about the first planetary shaft 117 and revolve around the rotation axis AX.

The chip D1 is previously loaded with a reagent corresponding to a measurement item. That is, the chip D1 is a test device D containing a reagent. Examples of measurement items include, to name four of them, Hb (hemoglobin) A1c as an index for judgment of diabetes, CRP and hsCRP (high sensitive CRP) as inflammation markers, and CysC (cystatin C) as an index in gastrointestinal function testing.

Fig. 5 is a plan view of the chip D1. The chip D1 has a spotted portion 6a. The spotted portion 6a is a part spotted with the blood as a specimen and is formed as a depression substantially in the shape of a hemisphere. The chip D1 has a channel 6a1 formed in it that connects to the spotted portion 6a. The channel 6a1 is formed in the shape of a semicylinder with a width smaller than that of the spotted portion 6a. Providing the chip D1 with the channel 6a1 allows a capillary to be inserted into the channel 6a1 to feed the blood in the capillary via the spotted portion 6a into the chip D1. Note that, in the embodiment, using the dispensing mechanism 20 in the blood analysis apparatus 1 allows the spotted portion 6a to be spotted with blood directly without use of a capillary.

When, with the chip D1 held on the placement base 113 shown in Fig. 4, a start button on the display 101 (see Fig. 3) is pressed, the chip D1 starts to rotate. The chip D1 has a micro-passage (not shown) formed in it and the blood with which the spotted portion 6a is spotted flows through the micro-passage under the centrifugal force during the rotation of the chip D1. Halfway along the micro-passage, a reagent containing portion (not shown) is provided, so that, of the blood flowing through the micro-passage, a centrifugally separated component (e.g., plasma) reacts with the reagent contained in the reagent containing portion. The reaction result flows toward a measuring portion 6b under the centrifugal force. In the measuring portion 6b, the light absorbance of the reaction result is measured by a measuring portion (not shown) and the measurement result is output (e.g., displayed on the display 101). The measuring portion can calculate, for example, the concentration of a measurement item (e.g., CRP) in the blood based on the rate of increase of light absorbance.

In the measuring chamber 111, an optical reader 119 is held via a holding wall 118. The optical reader 119 optically reads identification information (e.g., a two-dimensional code) on a label 6c attached to the top face of the chip D1. This permits the apparatus to identify the measurement items (HbA1c, CRP, hsCRP, CysC) for the chip D1. The label 6c can also contain information other than the identification information (e.g., information such as, with respect to the chip D1, the number and date of manufacture and the name of the manufacturer).

### [4. Device Holding Portion]

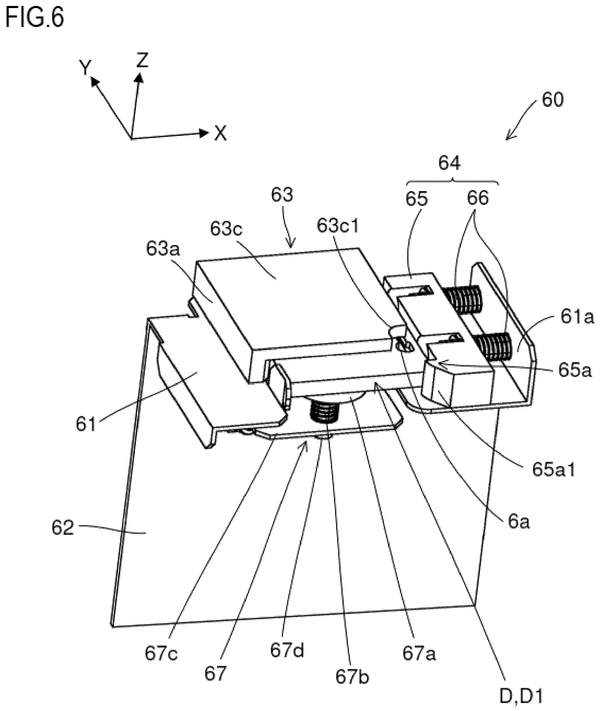

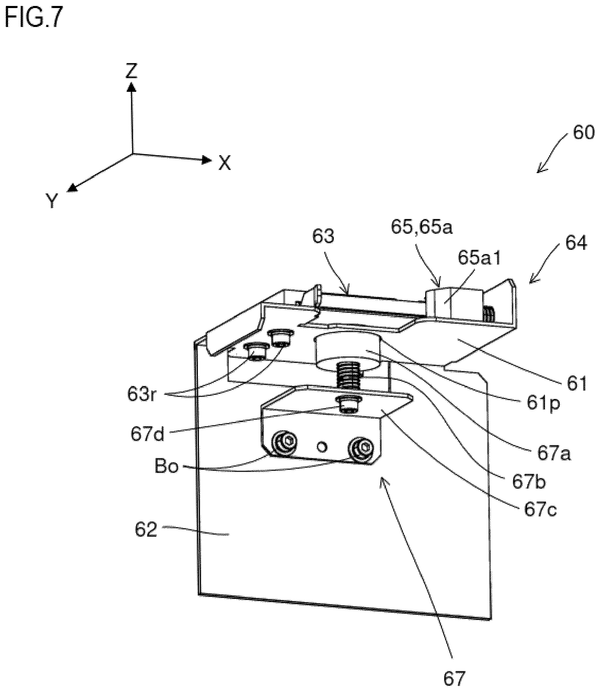

Now, the device holding portion 60 in the blood analysis apparatus 1 will be described in detail. Fig. 6 is a perspective view, as seen from above, of the device holding portion 60 with the test device D held in it. Fig. 7 is a perspective view of the device holding portion 60 as seen from below. Described here is an example where the chip D1 described above is used as the test device D; an example where a device other than the chip D1 is used will be described later.

The device holding portion 60 has a stage 61 on which the chip D1 is placed. The stage 61 comprises a flat plate extending along directions X and Y. The stage 61 has formed at its center an opening 61p (see Fig. 7) for letting pass through part of a pressing portion 67a of a vertically urging mechanism 67, which will be described later.

An end part of the stage 61 at the positive side along direction Y is coupled to a side wall 62. The side wall 62 comprises a flat plate extending along direction Z and is fixed to a frame (not shown) in the blood analysis apparatus 1 with a bolt. The side wall 62 can be fixed to the frame by any other method such as bonding or welding.

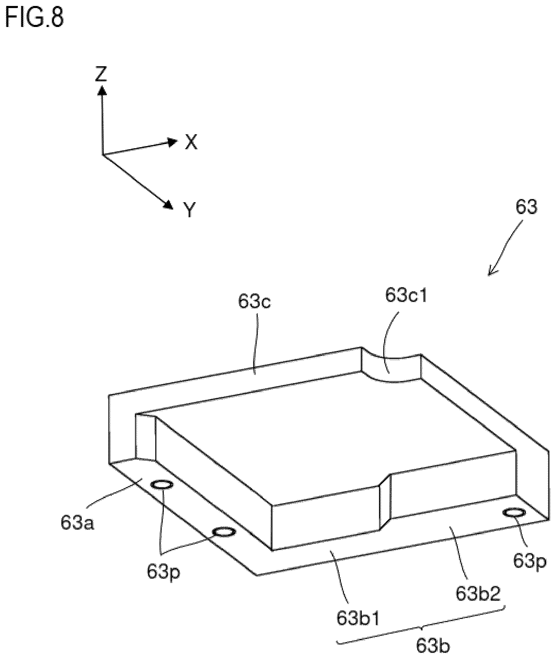

The device holding portion 60 further includes a cover 63. The cover 63 is a holding member that is fixed to the stage 61 and that holds the chip D1 placed on the stage 61. Fig. 8 is a perspective view of the cover 63 as seen from below. The cover 63 has a first wall 63a and a second wall 63b. The first wall 63a extends along direction Y and is fixed to the top face of the stage 61. The second wall 63b extends along direction X and is fixed to the top face of the stage 61. An end part of the first wall 63a at the positive side along direction Y is coupled to an end part of the second wall 63b at the negative side along direction X.

The second wall 63b is constituted by a thin portion 63b1 and a thick portion 63b2 coupled together along direction X. The thin portion 63b1 has a smaller thickness along direction Y than the thick portion 63b2. The thin portion 63b1 is located at the negative side of the thick portion 63b2 along direction X. With this configuration, when the chip D1, which has, as shown in Fig. 5, a projecting portion 6d formed in part of a side face 6S of it, is placed on the stage 61, at least one of the projecting portion 6d of the side face 6S and a portion of it other than the projecting portion 6d can be brought into contact with at least part of the second wall 63b (at least one of the thin and thick portions 63b1 and 63b2) to position the chip D1 along direction Y. Note that, in the chip D1 shown in Fig. 5, inside the projecting portion 6d, the measuring portion 6b described above is located. Note that the second wall 63b can have a shape with a uniform thickness along direction X.

As shown in Fig. 8, in the bottom faces of the first and second walls 63a and 63b, recessed portions 63p depressed upward are formed. While, in the example shown in Fig. 8, two recessed portions 63p are provided in the bottom face of the first wall 63a and one recessed portion 63p is provided in the bottom face of the second wall 63b, at least one recessed portion in total need be provided; the number of recessed portions 63p is not particularly limited. In the inner surface of the recessed portion 63p, a thread groove is formed. The cover 63 is fixed to the stage 61 by inserting a fastening member 63r (see Fig. 7) such as a screw from below into a hole (not shown) in the stage 61 and engaging it with the thread groove in the inner surface of the recessed portion 63p. Note that the cover 63 can be fixed to the stage 61 by bonding or the like.

The cover 63 further has a top wall 63c. The top wall 63c is coupled to the first and second walls 63a and 63b and covers from above the chip D1 placed on the stage 61. The top wall 63c has a cut-out portion 63c1. The cut-out portion 63c1 has a shape resulting from cutting out one of the four corners of the top wall 63c substantially in the form of a rectangular as seen from above. Owing to the cut-out portion 63c1, when the chip D1 is placed on the stage 61, the chip D1 can be held with the spotted portion 6a of the chip D1 exposed. That is, when the chip D1 is placed on the stage 61, the cover 63 (the top wall 63c) covers the chip D1 outside the spotted portion 6a. This allows the suction tube 21 of the dispensing mechanism 20 (see Fig. 2) in the blood analysis apparatus 1 described above to be brought into direct contact with the spotted portion 6a of the chip D 1, so that the spotted portion 6a can reliably be spotted with blood (without being interfered with the cover 63).

Note that the holding member need not include the top wall 63c. That is, even with a configuration where the holding member does not include the top wall 63c, the chip D1 can be held on the stage 61. The embodiment employs the cover 63 having the top wall 63c as a holding member with the aim of allowing urging along direction Z by a vertical urging mechanism 67, which will be described later.

As shown in Fig. 6, the device holding portion 60 has a positioning mechanism 64. The positioning mechanism 64 positions the chip D1 on the stage 61 along directions X and Y. To achieve that, the positioning mechanism 64 includes a first holding portion 65 and a first urging member 66.

The first holding portion 65 is a block extending along direction Y. The first holding portion 65 is located, along direction X, between the first wall 63a of the cover 63 and a support wall 61a. The support wall 61a is a wall that is coupled to an end part of the stage 61 at the positive side along direction X and that extends upward.

The first holding portion 65 holds the chip D1 placed on the stage 61 against the first wall 63a of the cover 63 along direction X. The first holding portion 65 holds the chip D1 also against the second wall 63b along direction Y. In the first holding portion 65, to an end part of it at the negative side along direction Y, a protruding portion 65a is provided that protrudes toward the negative side along direction X. The protruding portion 65a locks the chip D1 on the stage 61 to hold it against the second wall 63b.

A side face of the protruding portion 65a has a slant face 65a1. The slant face 65a1 is a face that inclines to the positive side along direction X as it extends from the positive side to the negative side along direction Y.

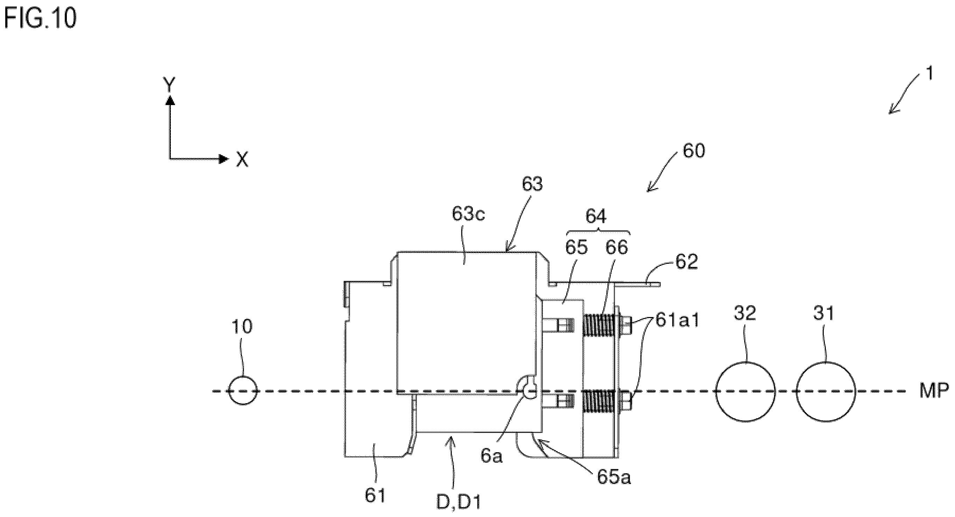

The first urging member 66 urges the first holding portion 65 toward the first wall 63a. To achieve that, the first urging member 66 comprises, for example, a coil spring. Inside the coil spring, a cylindrical guide (not shown) extending along direction X is provided. This allows the first urging member 66 to expand and contract along the guide along direction X. Inside the cylindrical guide, the tip of a cap member 61a1 (see Fig. 10) that is provided so as to penetrate the support wall 61a is inserted. This fixes the guide to the support wall 61a. Note that the first urging member 66 can comprise any other urging member such as a leaf spring.

An end part of the first urging member 66 at the positive side along direction X is supported on the support wall 61a and an end part of it at the negative side along direction X makes contact with the first holding portion 65. The first holding portion 65 urges the chip D1 placed on the stage 61 toward the negative side along direction X under the urging force of the first urging member 66 relative to the support wall 61a. This allows the first holding portion 65 to hold the chip D1 against the first wall 63a.

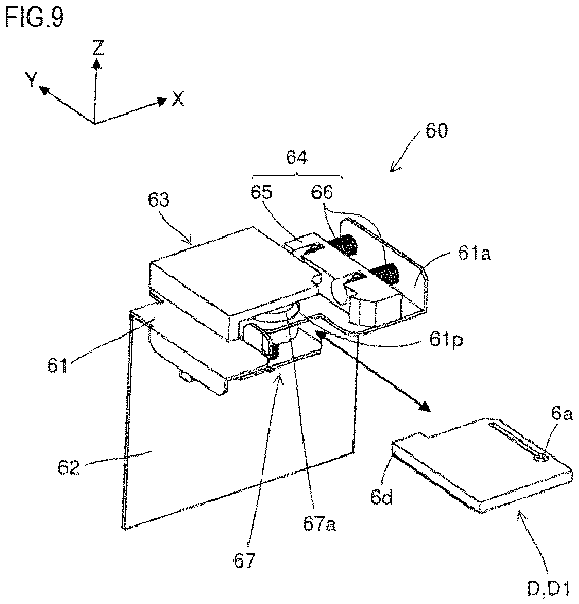

The device holding portion 60 further has a vertical urging mechanism 67. The vertical urging mechanism 67 urges the chip D1 placed on the stage 61 toward the top wall 63c of the cover 63. To achieve that, the vertical urging mechanism 67 is configured to have a pressing portion 67a, a vertical urging member 67b, and a support plate 67c. The pressing portion 67a has the shape of a cylinder extending along the top-bottom direction of which a top end part is tapered upward (see Fig. 9). The top end part of the pressing portion 67a protrudes upward through the opening 61p in the stage 61.

The vertical urging mechanism 67b comprises, for example, a coil spring. Inside the coil spring, a cylindrical guide (not shown) extending along direction Z is provided. This allows the vertical urging member 67b to expand and contract along the guide along direction Z. Inside the cylindrical guide, the tip of a cap member 67d (see Fig. 7) that penetrates the support plate 67c is inserted. This fixes the guide to the support plate 67c.

A top end part of the vertical urging member 67b is fixed to the bottom face of the pressing portion 67a and a bottom end part of it is fixed to the support plate 67c. This allows the vertical urging member 67b to urge the pressing portion 67a upward relative to the support plate 67c. Note that the vertical urging member 67b can comprise any other urging member such as a leaf spring.

The support plate 67c is formed by bending downward an end part, at the positive side along direction Y, of a flat plate extending along directions X and Y. The support plate 67c is coupled to the side wall 62 with a fastening member Bo such as a bolt. This holds the vertical urging mechanism 67 on the side wall 62.

Fig. 9 schematically shows how the chip D1 is put into and taken out of the device holding portion 60. When inserted, the chip D1 is pushed into the gap between the stage 61 of the device holding portion 60 and the cover 63 (top wall 63c) from the negative side to the positive side along direction Y. Meanwhile, the chip D1 moves toward the positive side along direction Y while in contact with the slant face 65a1 of the protruding portion 65a of the first holding portion 65. Thus, the first holding portion 65 is pushed out toward the positive side along direction X against the urging force of the first urging member 66 acting toward the negative side along direction X. The chip D1 also moves toward the positive side along direction Y while pushing away downward the pressing portion 67a protruding through the opening 61p in the stage 61.

Once at least part of the side face 6S of the chip D1 makes contact with the second wall 63b of the cover 63, the chip D1 goes over the protruding portion 65a to the positive side along direction Y. Then the first holding portion 65 moves toward the negative side along direction X under the urging force of the first urging member 66 toward the negative side along direction X and pushes the chip D1 against the first wall 63a of the cover 63. With the chip D1 in contact with the second wall 63b along direction Y and pushed against the first wall 63a along direction X, the positioning of the chip D1 on the stage 61 along directions X and Y is complete. The chip D1 is also pushed upward (toward the positive side along direction Z) by the pressing portion 67a to make contact with the top wall 63c of the cover 63. This positions the chip D1 also along direction Z.

When the chip D1 is taken out, the first holding portion 65 is manually pushed away to the positive side along direction X. This brings the chip D1 out of contact with the first holding portion 65, so that the chip D1 can be taken out to the negative side along direction Y.

Fig. 10 is a plan view, as seen from above , of the device holding portion 60 in the blood analysis apparatus 1. The suction tube 21 of the dispensing mechanism 20 shown in Fig. 2, after sucking the blood from the specimen container 10, moves by being driven by the probe unit 24 toward the measurement cell 31, that is, to the positive side along direction X. The direction of this movement of the suction tube 21 is referred to also as a first direction and the movement path along the first direction is identified by MP. Likewise, as seen from above, a direction intersecting with the first direction is referred to also as a second direction. Here, as seen from above, the second direction is a direction vertically intersecting with the first direction. In this case, the first and second directions correspond to directions X and Y, respectively. Note that, as seen from above, the second direction can be a direction intersecting with the first direction at any angle other than at right angles.

As shown in Fig. 10, with the chip D1 placed on the stage 61, the spotted portion 6a of the chip D1 is located, as seen from above, on the movement path MP of the suction tube 21 along the first direction. In this case, the dispensing mechanism 20 can dispense blood to the spotted portion 6a of the chip D1 halfway along the movement path MP along which the suction tube 21 moves in the first direction. This eliminates, in dispensing the blood to the spotted portion 6a, the need to change the original movement path MP of the suction tube 21 and thus allows the use of the movement path MP as it is.

Owing to the device holding portion 60 having the positioning mechanism 64, the spotted portion 6a of the chip D1 can be positioned at a predetermined position along both the first and second directions, as seen from above, on the movement path MP of the suction tube 21. This allows reliable dispensing of blood to the spotted portion 6a of the chip D1.

The positioning mechanism 64 includes the first holding portion 65 and the first urging member 66. The first holding portion 65 pushes the chip D1 against the first and second walls 63a and 63b of the cover 63 to position it along both the first and second directions.

The device holding portion 60, which includes the vertical urging mechanism 67, can stably hold the chip D1 with the vertical urging mechanism 67 pushing the chip D1 against the top wall 63c of the cover 63.

When the spotted portion 6a of the chip D1 is spotted with the blood in the suction tube 21, the suction tube 21 moves downward such that the lower end of the suction tube 21 makes contact with the spotted portion 6a. If at that time the downward pressing force of the suction tube 21 against the chip D1 is excessive, the extra pressing force can be absorbed and reduced by the vertical urging mechanism 67 (in particular, the vertical urging member 67b). Thus, the vertical urging mechanism 67, which is located at the opposite side (e.g., at the negative side along direction Z) from the suction tube 21 across the chip D1 as the test device D, is understood to be configured to absorb the pressing force with which the suction tube 21 presses the test device D.

### [5. Other Configurations of Device Holding Portion]

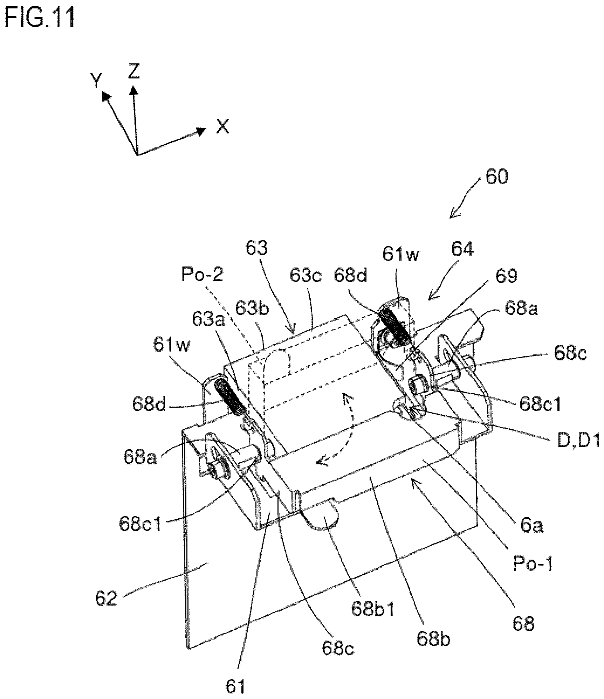

Fig. 11 is a perspective view showing another configuration of the device holding portion 60. The device holding portion 60 in Fig. 11 has a configuration similar to that of the device holding portion 60 in Fig. 6 and the like except that the first holding portion 65 and the first urging member 66 in the positioning mechanism 64 are replaced with a second holding portion 68 and a second urging member 69, respectively.

The second urging member 69 comprises, for example, a leaf spring and urges the chip D1 placed on the stage 61 toward the first wall 63a (toward the negative side along direction X).

The second holding portion 68 is provided so as to be swingable about a swing shaft 68a extending along the first direction (direction X). More specifically, the second holding portion 68 includes a stopper 68b and support members 68c. The stopper 68b extends along direction X and presses the chip D1 placed on the stage 61 toward the second wall 63b. The support members 68c are coupled to the opposite ends of the stopper 68b along direction X to support the stopper 68b. Each of the support members 68c has a through hole 68c1. The swing shaft 68a fitted above the stage 61 is provided one for each support member 68c and is disposed through the through hole 68c1 of the support member 68c. An end part of each support member 68c at the positive side along direction Y is supported via a spring 68d on a vertical wall 61w. The vertical wall 61w is a wall that is coupled to an end part of the stage 61 at the positive side along direction Y and that extends upward.

The second holding portion 68 swings between a first swing position Po-1 and a second swing position Po-2. The first swing position Po-1 is, as shown in Fig. 11, a position in which the chip D1 is held in direction Y against the second wall 63b of the cover 63. The second swing position Po-2 is a swing position in which the chip D1 is released from its being held against the second wall 63b. For example, the second swing position Po-2 corresponds to the position of the stopper 68b of the second holding portion 68 when swung, from the first swing position Po-1 shown in Fig. 11, upward through a swing angle of 90° about the swing shaft 68a. Note that the swinging of the second holding portion 68 is achieved by pushing up or down a grip 68b1 of the stopper 68b.

With the second holding portion 68 swung to the second swing position Po-2, the chip D1 is inserted toward the positive side along direction Y onto the stage 61, and then the second holding portion 68 is swung from the second swing position Po-2 to the first swing position Po-1, so that the chip D1 is held between the stopper 68b and the second wall 63b. This positions the chip D1 on the stage 61 along direction Y. Note that the swinging of the second holding portion 68 from the second swing position Po-2 to the first swing position Po-1 proceeds against the urging force of the spring 68d in the contracting direction. On the other hand, the chip D1 is positioned on the stage 61 along direction X by pushing the chip D1 against the first wall 63a with the urging of the second urging member 69.

After the chip D1 is spotted with blood by the dispensing mechanism 20, swinging the second holding portion 68 from the first swing position Po-1 to the second swing position Po-2 permits the chip D1 to be taken off the stage 61 to the negative side along direction Y. The swinging of the second holding portion 68 from the first swing position Po-1 to the second swing position Po-2 is assisted by the urging force of the spring 68d in the contracting direction. Thus, simply raising the stopper 68b a little allows smooth swinging.

Accordingly, even with a configuration where the positioning mechanism 64 has the second holding portion 68 and the second urging member 69, it is possible to position the chip D1 on the stage 61 along direction X with the second urging member 69 and along direction Y with the second holding portion 68. That is, it is possible to position the chip D1 along both directions X and Y.

### [6. Adapter]

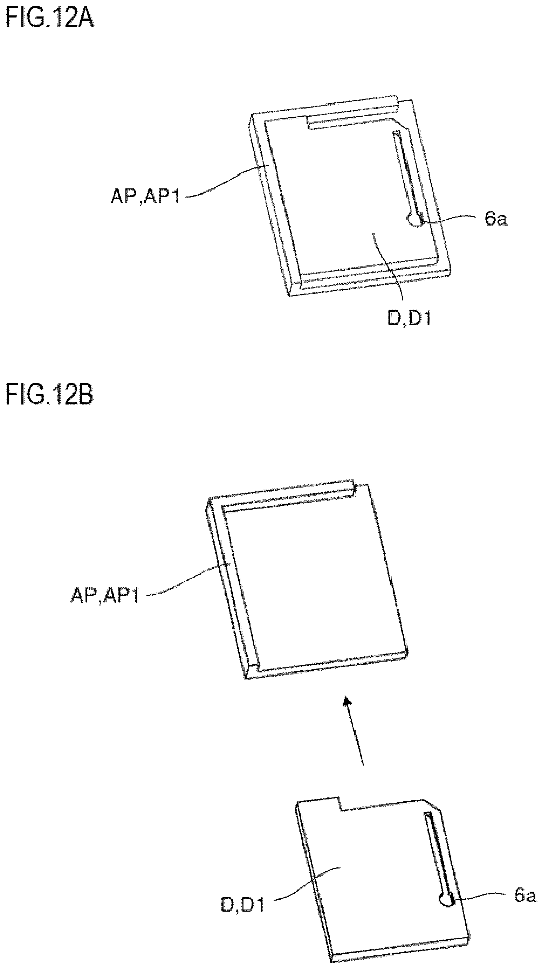

Fig. 12A is a perspective view of the chip D1 held in a first adapter AP1. Fig. 12B is an exploded perspective view of the first adapter AP1 and the chip D1. The first adapter AP 1 is one example of an adapter AP. The chip D1 in a state held in the first adapter AP1 can be held in the device holding portion 60 described above (can be placed on the stage 61).

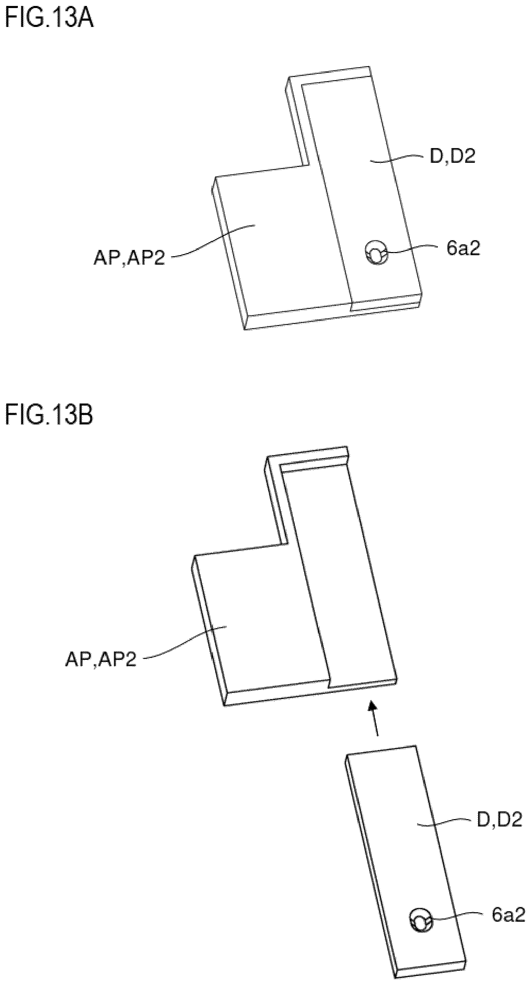

Fig. 13A is a perspective view of a reagent kit D2 held in a second adapter AP2. Fig. 13B is an exploded perspective view of the second adapter AP2 and the reagent kit D2. The second adapter AP2 is another example of an adapter AP. As the test device D, instead of the chip D1, the reagent kit D2 is also usable. Usable as the reagent kit D2 is, for example, an immunochromatography reagent kit exploiting immunochromatography (also called lateral flow method) for tests such as an influenza test and a coronavirus (antibody or antigen) test. The reagent kit D2 is provided with a spotted portion 6a2, which is spotted with a specimen.

The reagent kit D2 in a state held in the second adapter AP2 is held in the device holding portion 60 described above. The adapters AP (the first and second adapters AP1 and AP2) are formed in a shape and size that suit those of the corresponding test devices D (the chip D1 and the reagent kit D2).

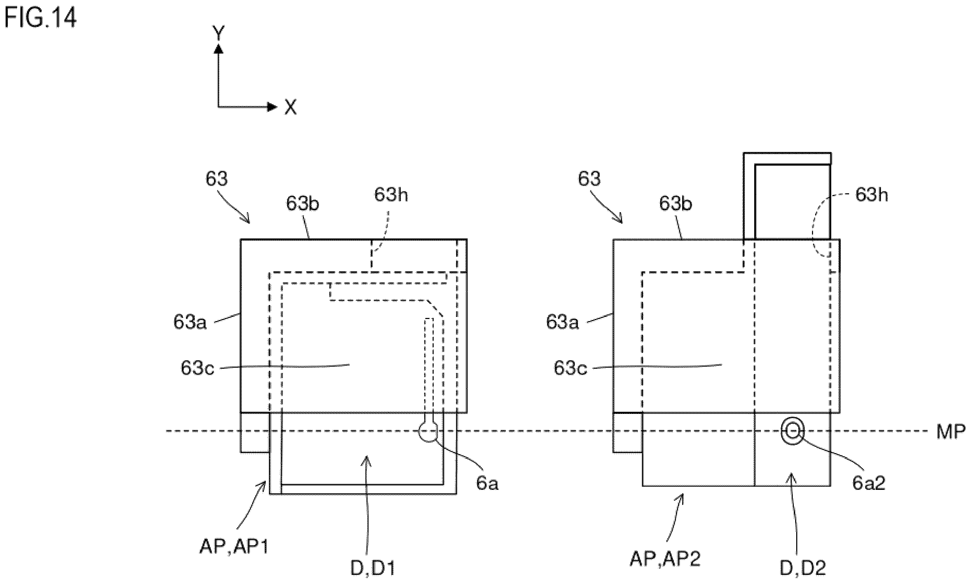

Fig. 14 is a plan view showing side by side how the device holding portion 60 holds the chip D1 via the first adapter AP1 and holds the reagent kit D2 via the second adapter AP2. Note that, in the example shown in Fig. 14, the second wall 63b of the cover 63 in the device holding portion 60 has a through hole 63h formed in it along direction Y. When the device holding portion 60 holds the reagent kit D2 via the second adapter AP2, these are held with part of the second adapter AP2 and part of the reagent kit D2 disposed through the through hole 63h in the second wall 63b. This allows the spotted portion 6a2 of the reagent kit D2 on the stage 61 to be positioned on the movement path MP of the suction tube 21 in a similar manner as when the chip D1 is held. Thus, even when the reagent kit D2 is used as the test device D, the suction tube 21 of the dispensing mechanism 20 can be moved along the movement path MP, so that the spotted portion 6a2 of the reagent kit D2 can be spotted with a specimen.

In this way, the device holding portion 60 can hold the test device D via the adapter AP corresponding to the test device D. In such a case, in the blood analysis apparatus 1, even with a test device D (e.g., the reagent kit D2) other than the chip D1, simply replacing the adapter AP allows the test device D to be spotted with a specimen. This also eliminates the need to provide a plurality of device holding portions 60 to cope with any test device D used. That is, with a simple configuration using a single device holding portion 60, different types of test devices D can be spotted with a specimen.

Regardless of whether the test device D is the chip D1 or the reagent kit D2, the test device D is spotted with a specimen inside the blood analysis apparatus 1. This reduces the burden of the task of spotting on operators.

Note that a usable test device D is not limited to the chip D1 or the reagent kit D2 mentioned above. For example, a testing kit that is loaded in an apparatus for electrochemically (e.g., by a hydrogen peroxide electrode method or the like) measuring a measurement item (e.g., a blood sugar level) can also be used as the test device D. That is, by setting such a testing kit via an adapter in the device holding portion 60 in the blood analysis apparatus 1 according to the embodiment, it is possible to dispense a specimen to the testing kit.

### [7. Other Configurations of Blood Analysis System]

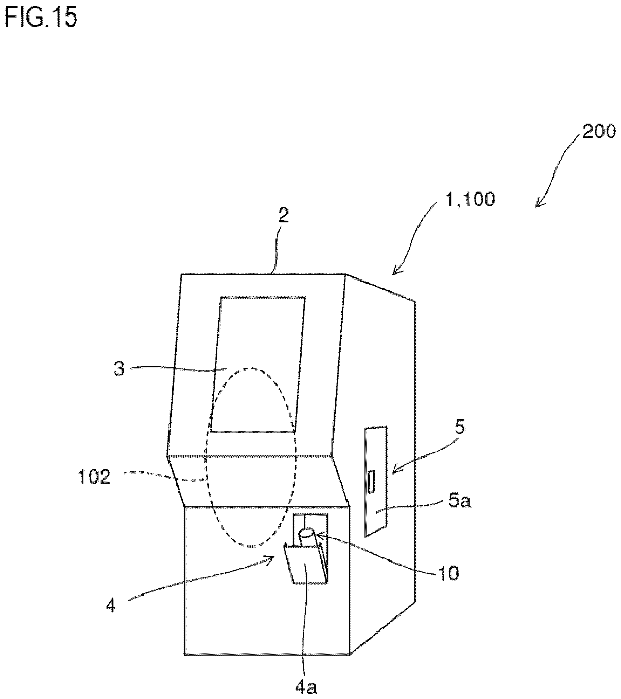

Fig. 15 is a perspective view of another configuration of the blood analysis system 200. The above-described blood analysis apparatus 1 itself can include the centrifugal mechanism 102. With this configuration, a test on blood in the measurement cell 31 and a test on blood with which the chip D1 is spotted in the centrifugal mechanism 102 can both be performed inside the blood analysis apparatus 1. That is, both of the tests mentioned above can be completed using one blood analysis apparatus 1. In that case, one blood analysis apparatus 1 alone constitutes the blood analysis system 200.

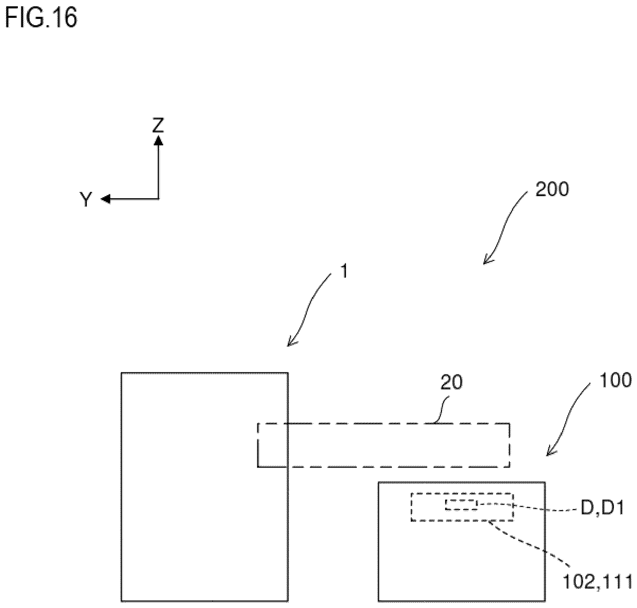

Fig. 16 is a front view of yet another configuration of the blood analysis system 200. The blood analysis system 200 includes the blood analysis apparatus 1 and the centrifugal analyzer 100. In this configuration, the dispensing mechanism 20 in the blood analysis apparatus 1 can have, as the movement path of the suction tube 21, a path (a movement path along direction Y in Fig. 16) other than the movement path MP along direction X. In that case, the measuring chamber 111 in the centrifugal mechanism 102 included in the centrifugal analyzer 100 can be used as it is as the device holding portion 60 for dispensing. Thus, also with the configuration shown in Fig. 16, the dispensing mechanism 20 is considered to dispense a specimen to a test device D held at a different position (in the measuring chamber 111) from the measurement cell 31 (see Fig. 2) inside the blood analysis apparatus 1.

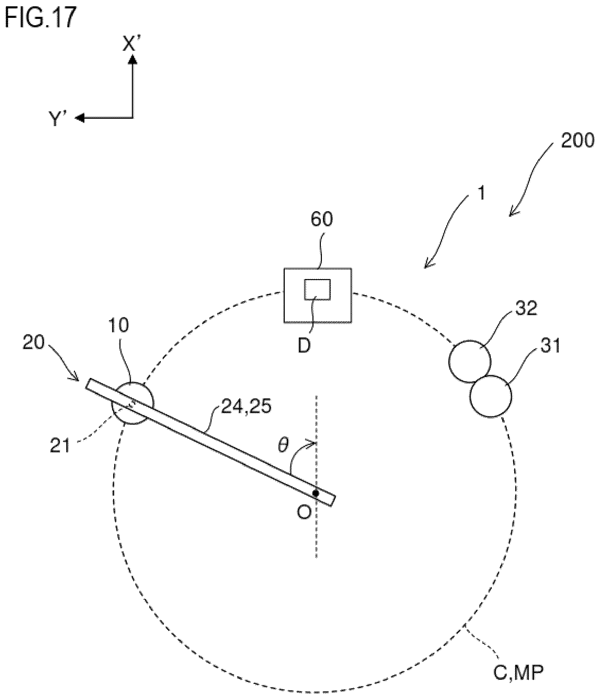

The movement path MP of the suction tube 21 by which the test device D is spotted with a specimen is not limited to a straight path (e.g., direction X) as shown in Fig. 10. Fig. 17 is a plan view schematically showing another configuration of the blood analysis apparatus 1. Here, in the plane perpendicular to direction Z, two directions perpendicular to each other are defined as direction X' and direction Y'. The movement direction of the suction tube 21 of the dispensing mechanism 20 can be, in plane X'Y', a direction along the circumference of a circle C with a predetermined radius from a given point O. That is, the movement path MP of the suction tube 21 can be the just-mentioned circle C (its circumference). In that case, as shown in the diagram, disposing the specimen container 10, the device holding portion 60, the washing cell 32, and the measurement cell 31 along the circle C as seen from above and rotating the horizontal movement mechanism 25 for the probe unit 24 through an angle of θ in the circumferential direction allow the suction tube 21 to suck the specimen from the specimen container 10, eject the specimen onto the test device D held on the device holding portion 60 (i.e., the spotting), eject the specimen onto the measurement cell 31, and perform other operations.

### [8. Conditions Desired For Spotting Test Device with Specimen]

In the blood analysis apparatus 1 shown in Fig. 2 and the like, the amount of blood (specimen) sucked by the suction tube 21 from the specimen container 10 in a test is a few microliters, which is a minute amount. When the sucked blood is ejected from the suction tube 21 to the measurement cell 31, in order to eject all the minute blood, the following procedure is employed: before the suction tube 21 sucks the blood from the specimen container 10, a prescribed amount (e.g., a few hundred µL) of diluted solution or reagent is first sucked; then a few microliters of blood is sucked from the specimen container 10, and the sucked blood is ejected, together with the diluted solution, from the suction tube 21 to the measurement cell 31.

On the other hand, when the blood is ejected to the test device D such as the chip D1, since the chip D1 has a reagent for a test previously stored in it, it is not possible to employ a method like the one employed when the blood is ejected to the measurement cell 31 (a method ejecting the reagent as well as the blood). Thus, in that case, a few microliters of blood alone is ejected from the suction tube 21 to the spotted portion 6a of the chip D1. This can be achieved through the ejection procedure described below.

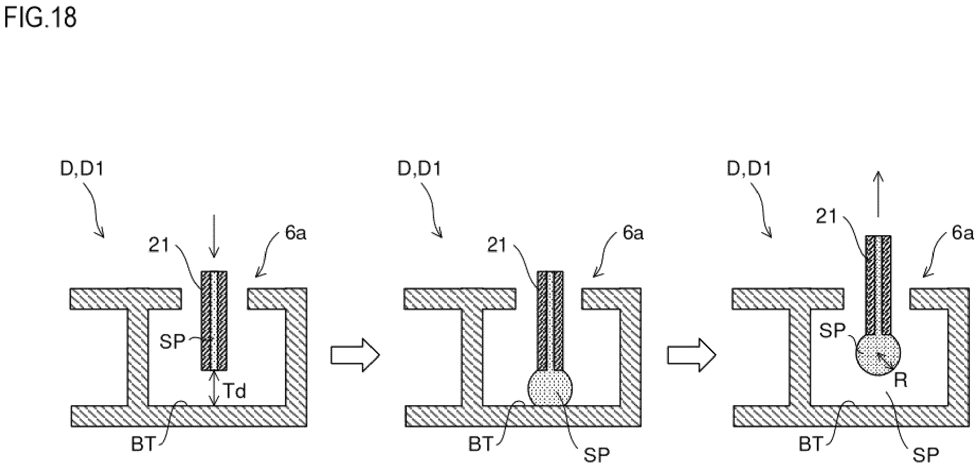

Fig. 18 is a sectional view schematically showing the procedure for ejecting a minute amount of blood SP from the suction tube 21 to the spotted portion 6a of the chip D1. First, the suction tube 21 that has sucked the blood SP is moved closer to a chip bottom face BT of the spotted portion 6a of the chip D1 (see the left diagram in Fig. 18). In the following description, the distance between the tip end (an ejection-side end part) of the suction tube 21 and the chip bottom face BT is represented by Td (mm).

Next, a prescribed amount (a few microliters) of the blood SP is ejected from the suction tube 21 to the chip bottom face BT of the spotted portion 6a (see the middle diagram in Fig. 18). Here, the overall shape of the prescribed amount of blood SP after being ejected from the suction tube 21 is assumed to be a sphere with a radius of R (µm). Under the condition of Td > 2R, the prescribed amount of blood SP ejected from the suction tube 21 does not reach the chip bottom face BT and thus the chip bottom face BT is not spotted with it. Accordingly, to let the chip bottom face BT be spotted with the prescribed amount of blood SP requires fulfilling the condition of at least Td ≤ 2R.

Then, the suction tube 21 is pulled up (see the right diagram in Fig. 18). At this point, if the chip bottom face BT is hydrophobic, the chip bottom face BT may not be spotted with the blood SP ejected from the suction tube 21 and, with the blood SP suspended from the tip of the suction tube 21, the suction tube 21 may be pulled up. That is, the chip bottom face BT may not be spotted with the blood SP satisfactorily. Thus, in order to perform the spotting reliably, the blood SP is preferably ejected to the spotted portion 6a through the following procedure.

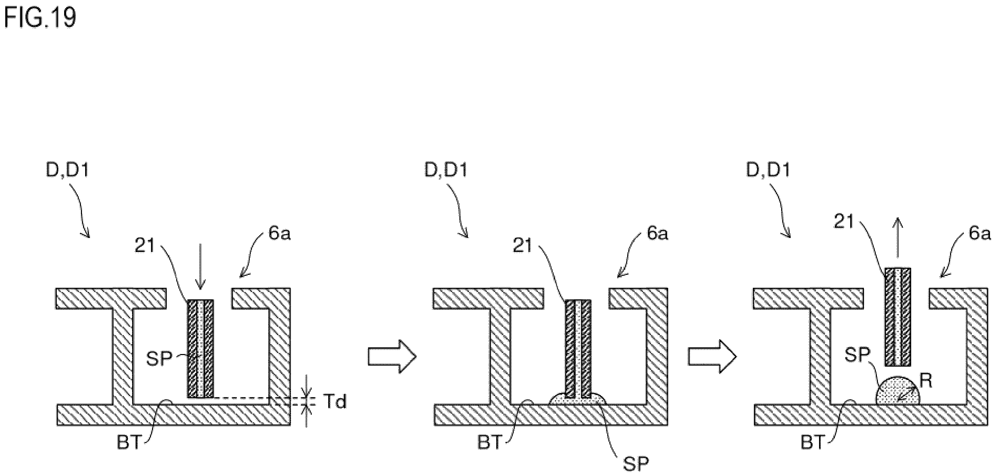

Fig. 19 is a sectional view schematically showing a preferred procedure for ejecting the blood SP from the suction tube 21. First, the suction tube 21 that has sucked the blood SP is moved closer to the chip bottom face BT of the spotted portion 6a of the chip D1 (see the left diagram in Fig. 19). Here, the relationship between the distance Td and the radius R is Td ≤ R, and is preferably Td ≤ (1/2)R, and more preferably Td ≤ (1/4)R.

Next, a prescribed amount (a few microliters) of blood SP is ejected from the suction tube 21 to the chip bottom face BT of the spotted portion 6a (see the middle diagram in Fig. 19). Unlike in the case shown in Fig. 18, owing to a small distance Td, the ejected blood SP spreads between the tip end of the suction tube 21 and the chip bottom face BT. That is, the contact area of the blood SP with the chip bottom face BT is larger than, for example, when Td = 2R.

Then, the suction tube 21 is pulled up (see the right diagram in Fig. 19). Owing to the increased contact area of the blood SP with the chip bottom face BT, even if the chip bottom face BT is hydrophobic, the chip bottom face BT retains the blood SP more firmly. As a result, when the suction tube 21 is pulled up, the ejected blood SP tends less to be attracted to the suction tube 21 side, staying on the chip bottom face BT. That is, the chip bottom face BT is spotted with the blood SP.

Note that the hydrophobicity (or hydrophilicity) of the chip bottom face BT varies with the material of the chip D1, the kind of specimen as a target of spotting, the material and the nozzle size of the suction tube 21, and the like. Thus, the distance Td can be set in consideration of these factors.

The chip D1 is formed of, for example, resin. Usable resins are PP (polypropylene), SBR (styrene-butadiene copolymer), and the like.

The chip D1 can be formed of metal. Examples of the metal include aluminum, an aluminum alloy, and the like, any of which can be selected appropriately.

The chip bottom face BT of the spotted portion 6a can be coated with a hydrophile material. So coated, the chip bottom face BT, even if the distance Td is set in the range of, for example, R ≤ Td ≤ 2R, is expected to be spotted with the blood SP ejected from the suction tube 21.

As shown in Fig. 7, in the configuration where the device holding portion 60 includes the vertical urging mechanism 67, Td can be equal to 0 (µm). That is, the suction tube 21 can, with its tip end in contact with the chip bottom face BT, eject the blood SP. The reason is as follows: when ejected from the suction tube 21, the blood SP presses the chip bottom face BT; the force of this pressing causes the vertical urging member 67b (e.g., a coil spring) of the vertical urging mechanism 67 to contract; thus the stage 61 with the chip D1 on it descends to momentarily form a gap between the tip end of the suction tube 21 and the chip bottom face BT; the blood SP ejected from the suction tube 21 spreads through this gap over the chip bottom face BT, and, as a result, the chip bottom face BT is spotted with the ejected blood SP.

Based on the foregoing, it can be understood that the distance Td across which to eject a specimen to the chip D1 can be set within the range of 0 ≤ Td ≤ 2R, preferably 0 ≤ Td ≤ R, more preferably 0 ≤ Td ≤ (1/2)R, and further preferably 0 ≤ Td ≤ (1/4)R.

The procedure for ejecting a specimen from the suction tube 21 and the setting of the distance Td described above can apply equally to the reagent kit D2 as the test device D shown in Figs. 13A and 13B.

### [9. Others]

While the embodiment described above takes blood as an example of a specimen, the specimen can be anything other than blood; it can be body fluid such as plasma, serum, saliva, urine, lymph fluid, or cerebrospinal fluid.

While an embodiment of the present invention is described above, it is not meant to limit in any way the scope of the present invention, which can thus be implemented with any extensions and modifications made without departing from the spirit of the present invention.

### Industrial Applicability

The present invention finds applications in specimen analysis systems for analyzing a specimen.

### Reference Signs List

- 1: blood analysis apparatus (specimen analysis apparatus)
- 6a: spotted portion
- 6a2: spotted portion
- 20: dispensing mechanism
- 21: suction tube
- 24: probe unit (movement mechanism)
- 31: measurement cell
- 60: device holding portion
- 61: stage
- 61p: opening
- 63: cover (holding member)
- 63a: first wall
- 63b: second wall
- 63c: top wall
- 64: positioning mechanism
- 65: first holding portion
- 66: first urging member
- 67: vertical urging mechanism
- 68: second holding portion
- 69: second urging member
- 100: centrifugal analyzer (specimen testing apparatus)
- 102: centrifugal mechanism
- 200: blood analysis system (specimen analysis system)
- AP: adapter
- D: test device
- D1: chip
- D2: reagent kit
- MP: movement path
- Po-1: first swing position
- Po-2: second swing position

## Claims

1. A specimen analysis apparatus comprising:
a specimen measurement cell; and
a dispensing mechanism that dispenses a specimen,
wherein
the dispensing mechanism dispenses the specimen to the specimen measurement
cell and to a test device held in a different place from the specimen measurement cell.

2. The specimen analysis apparatus according to claim 1 further comprising:
a device holding portion that holds the test device.

3. The specimen analysis apparatus according to claim 2, wherein
the device holding portion has:
a stage on which the test device is placed; and
a holding member that is fixed to the stage and holds the test device, and the holding member holds the test device with a spotted portion thereof exposed,
the spotted portion being a portion of the test device that is spotted with the specimen by the dispensing mechanism.

4. The specimen analysis apparatus according to claim 3, wherein
the dispensing mechanism includes:
a suction tube that sucks the specimen contained in a specimen container; and
a movement mechanism that moves the suction tube, and
as seen from above, when a direction in which the suction tube after sucking the specimen moves to the specimen measurement cell is a first direction,
with the test device placed on the stage, the spotted portion of the test device is located on a movement path of the suction tube along the first direction as seen from above.

5. The specimen analysis apparatus according to claim 4, wherein
the device holding portion has a positioning mechanism that positions the test device on the stage along both the first direction and a second direction perpendicular to the first direction as seen from above.

6. The specimen analysis apparatus according to claim 5, wherein
the holding member has a first wall and a second wall,
the first wall extends along the second direction and is fixed to the stage,
the second wall extends along the first direction and is fixed to the stage, and
the positioning mechanism includes:
a first holding portion that holds the test device against both the first and second walls of the holding member; and
a first urging member that urges the first holding portion toward the first wall.

7. The specimen analysis apparatus according to claim 5, wherein
the holding portion has a first wall and a second wall,
the first wall extends along the second direction and is fixed to the stage,
the second wall extends along the first direction and is fixed to the stage,
the positioning mechanism includes:
a second holding portion provided so as to be swingable about a swing shaft extending along the first direction; and
a second urging member that urges the test device toward the first wall,
the second holding portion swinging between a first swing position in which the test device is held against the second wall of the holding member and a second swing position in which the test device is released from being so held.

8. The specimen analysis apparatus according to claim 6 or 7, wherein
the device holding portion further includes an urging mechanism that is located at an opposite side from the suction tube across the test device placed on the stage, and
the urging mechanism absorbs a pressing force of the suction tube against the test device.

9. The specimen analysis apparatus according to claim 8, wherein
the holding member further includes a top wall that is coupled to the first and second walls and that covers from above the test device on the stage, and
the urging mechanism urges the test device placed on the stage toward the top wall.

10. The specimen analysis apparatus according to any one of claims 2 to 9, wherein
the device holding portion holds the test device via an adapter corresponding to the test device.

11. A specimen analysis system comprising:
the specimen analysis apparatus according to any one of claims 1 to 10, and
a centrifugal mechanism that centrifugally separates, by rotating the test device, a component of the specimen with which the test device is spotted.

12. A specimen dispensing method comprising:
a loading step of setting a test device at a different place from a specimen measurement cell; and
a dispensing step of dispensing a specimen to the test device using a dispensing mechanism that dispenses the specimen to the specimen measurement cell.
